# EUROPEAN PATENT APPLICATION

(11) **EP 4 397 256 A1**
(43) Date of publication of application: **10.07.2024**
(21) Application number: 22889486.1
(22) Date of filing: 08.11.2022
(51) Int. Cl.: A61B 17/32

(54) **CONSUMABLE MANAGEMENT SYSTEM AND MANAGEMENT METHOD AND VASCULAR CALCIFICATION TREATMENT DEVICE**

(30) Priority: 08.11.2021 CN 202111316464
(71) Applicant: Jiangsu Polylive Medtech Co., Ltd., Nantong, Jiangsu 226400 (CN)
(72) Inventor: TANG, Zhihuang, Nantong, Jiangsu 226400 (CN); LI, Chuang, Nantong, Jiangsu 226400 (CN)
(74) Representative: Zaboliene, Reda
(86) International application number: PCT/CN2022/130739
(87) International publication number: WO 2023/078465

(57) **Abstract**

A consumable end management system and management method and a vascular calcification treatment device. The consumable end management system comprises a control circuit (100) and a consumable circuit (200); the control circuit (100) comprises a processing module (110), a first power supply module (120), and a second power supply module (130); the consumable circuit (200) comprises a fusing module (210); the consumable circuit (200) is provided at a consumable end (20); the processing module (110) is configured to output a detection trigger signal to the first power supply module (120) and output, when the state of the fusing module (210) is determined to be a connected state, a fusing trigger signal to the second power supply module (130); the first power supply module (120) is configured to receive the detection trigger signal and output a first power supply signal to the fusing module (210) for state detection of the fusing module (210); the second power supply module (130) is configured to receive the fusing trigger signal and output a second power supply signal to the fusing module (210) so as to enable the fusing module (210) to be in a disconnected state. A disposable consumable end (20) can be distinguished and recognized, such that secondary use of consumables is effectively avoided, and safety risks possibly caused by secondary use are avoided. The present invention is high in stability and low in cost.

## Description

This application claims the priority of Chinese Patent Application No. 202111316464.X, entitled "CONSUMABLE MANAGEMENT SYSTEM, MANAGEMENT METHOD AND VASCULAR CALCIFICATION TREATMENT DEVICE", filed on November 8, 2021, the entire contents of which are incorporated by reference in this application.

### TECHICAL FIELD

The present application relates to the technical field of medical devices, and in particular to a consumable management system, a management method, and vascular calcification treatment device.

### BACKGROUND

Vascular stenosis refers to the pathological process in which hydroxyapatite minerals are deposited in the vascular system. Due to abnormal lipid metabolism in human arteriovenous, coronary, peripheral, and intracranial blood vessels, the lipids in the blood are deposited in the originally smooth blood vessels. The intima gradually accumulates into atherosclerotic lipid plaques. Over time, the lipid plaques increase and calcify, resulting in narrowing of the vessel lumen, obstructing blood flow, and causing ischemia in downstream vessels and the body, resulting in corresponding clinical manifestations. Among them, 30% to 50% of vascular stenosis will have calcified plaques, and both coronary arteries and peripheral arteries may have calcified lesions, which is extremely difficult to treat. The current interventional surgery for the treatment of calcified plaque is generally "plaque atherectomy/rotary atherectomy", but this surgical method is extremely difficult. Not only that, but rotary atherectomy can only deal with superficial calcification of intimal vessels, but is helpless for medial calcification, calcified nodules or severe calcification.

With the development of cardiovascular interventional technology, the techniques for targeting vascular calcified lesions are increasingly diversified. At present, it is attempted to use a shock wave treatment device consisting of a generator, a catheter, and a consumable balloon (a consumable electrode is arranged in the balloon), that is, a calcification treatment device. The balloon is placed in the lesion through the guide wire, and then the calcification is fragmented using ultrasonic technology to achieve the purpose of treatment. Generators and catheters can be used for patient treatment multiple times in a row, but the balloon is used as a disposable medical consumable, and the consumable balloon is in direct contact with the human body, so the safety of the consumable is also extremely important.

### Overview of the invention

### Technical Problem

The applicant found that, in general, the selection, use and postoperative failure treatment of consumables are all manually performed by the operator, and there is no corresponding failure treatment mechanism for consumables and device themselves. At present, the effective control of consumables is mainly carried out through human methods, and errors will inevitably occur in human control. During the human control, if the consumables are not correctly distinguished, the used disposable consumables are connected to the device again, and there will be great safety hazards in treating patients when the equipment cannot distinguish the use of consumables. On the one hand, with the use of consumables for treatment, there is loss of consumables. Excessive use of consumables will make it difficult to guarantee their internal reliability, which will pose a threat to the safety and health of patients. On the other hand, it is difficult to guarantee the sterilization safety of consumables during secondary use, and the use of incompletely sterilized consumables will pose a serious threat to the health of patients, which will inevitably lead to medical safety accidents.

### Solution To The Problem

### Technical Solution

In view of the above technical problems, this application provides a consumable management system, management method and vascular calcification treatment device, which can distinguish and identify a disposable consumable, so that a secondary use of a consumable can be effectively avoided, thereby avoiding the secondary use that may cause safety risk, high stability and low cost.

In order to achieve the above object, an embodiment of the present invention provides a consumable management system. As one of the embodiment, the consumable management system includes: a control circuit and a consumable circuit, and the control circuit includes a processing module and a first power supply module and a second power supply module, the consumable circuit includes a fusing module, the consumable circuit is arranged in a consumable, wherein,
the processing module is configured to output a detection trigger signal to the first power supply module, and output a fusing trigger signal to the second power supply module when determining that a state of the fusing module is a connected state;
the first power supply module is configured to receive the detection trigger signal and output a first power supply signal to the fusing module for a state detection of the fusing module;
the second power supply module is configured to receive the fusing trigger signal and output a second power supply signal to the fusing module for keeping the fusing module in a disconnected state.

In one embodiment, the processing module outputs the fusing trigger signal to the second power supply when determining that the state of the fusing module is a connected state and the processing module triggers pulse treatment, to control the second power supply module to output the second power supply signal to the fusing module, so as to keep the fusing module in a disconnected state.

In one embodiment, the fusing module includes a fuse and a resistor, the first end of the fuse is configured to receive the first power supply signal or the second power supply signal, and the second end of the fuse to ground through the resistor;

The processing module is connected to the second end of the fuse, and is configured to obtain a monitoring signal, so as to determine the state of the fusing module according to the monitoring signal.

In one embodiment, the first power supply module includes a first switch and a voltage source, and the second power supply module includes a second switch and a current source;
when the first switch receives the detection trigger signal, the first switch switches on the voltage source and the fusing module, so that the voltage source outputs the first power supply signal to the fusing module;
when the second switch receives the fusing trigger signal, the second switch switches on the current source and the fusing module, so that the current source outputs the second power supply signal to the fusing module.

In one embodiment, the control circuit further includes a third power supply module, the consumable circuit also includes a connection module, the connection module is connected to the third power supply module and the processing module through feedback signal fines; wherein,
when the processing module receives a feedback signal corresponding to a third power supply signal output by the third power supply module through the connection module, the processing module outputs the detection trigger signal to the first power supply module.

Based on the same inventive concept, the present invention provides a consumable management method. In one embodiment, the method includes:
outputting a detection trigger signal to a first power supply module, so that the first power supply module outputs a first power supply signal to a fusing module of a consumable;
obtaining a monitoring signal of the fusing module, to determine a state of the fusing module according to the monitoring signal;
when determining a state of the fusing module is a connected state, outputting a fusing trigger signal to a second power supply module, so that the second power supply module outputs a second power supply signal to the fusing module, and the fusing module fuses according to the second power supply signal.

In one embodiment, a step of outputting the detect trigger signal to the first power supply module so that the first power supply module outputs the first power supply signal to the fusing module of the consumable includes:
when receiving a feedback signal corresponding to a third power supply signal output by a third power supply module through the consumable, outputting the detection trigger signal to the first power supply module, so that the first power supply module outputs the first power supply signal to the fusing module of the consumable.

In one embodiment, a step of when determining the state of the fusing module is the connected state, outputting the fusing trigger signal to the second power supply module, so that the second power supply module outputs the second power supply signal to the fusing module, and the fusing module fuses according to the second power supply signal, includes:
when determining the state of the fusing module is connected state, monitoring a pulse treatment control signal;
when obtaining the pulse treatment control signal, outputting the fusing trigger signal to the second power supply module, so that the second power supply module outputs the second power supply signal to the fusing module, so that the fusing module fuses according to the second power supply signal.

In one embodiment, after a step of when obtaining the pulse treatment control signal, outputting the fusing trigger signal to the second power supply module, so that the second power supply module outputs the second power supply signal to the fusing module, so that the fusing module fuses according to the second power supply signal, includes:
outputting the detection trigger signal to the first power supply module, so that the first power supply module outputs the first power supply signal to the fusing module of the consumable;
obtaining a monitoring signal of the fusing module to determine the state of the fusing module according to the monitoring signal;
when determining that the fusing module is in a disconnected state, setting a remaining treatment number of the consumable to an effective maximum;
when the remaining treatment number of the consumable is greater than 0, and receiving the pulse treatment control signal, triggering pulse treatment and decreasing the remaining treatment number of the consumable;
when the remaining treatment number of the consumable is equal to 0 and receiving the pulse treatment control signal, not triggering the pulse treatment.

Based on the same inventive concept, the present invention also provides a vascular calcification treatment device. In one embodiment, the vascular calcification treatment device includes a device, a connector, and the consumable described in any of the above-mentioned embodiments, the device is connected to the consumable through the connector, the consumable also includes a consumable electrode, the device includes a main power supply module, a buck circuit, a booster circuit and the control circuit as described in any of the above-mentioned embodiments; wherein,
the main power supply module is connected to the booster circuit, and connected to the processing module, the first power supply module and the second power supply module through the buck circuit, to supply power;
the processing module is connected to the booster circuit, to control the booster circuit output a pulse signal;
the booster circuit is connected to the consumable electrode, to form a high voltage discharge loop to release the pulse signal.

### Beneficial Effect

To sum up, the consumable management system provided by the embodiment of the present invention includes a control circuit and a consumable circuit, the control circuit includes a processing module, a first power supply module, and a second power supply module, the consumable circuit includes a fusing module, and the consumable circuit is arranged in the consumable, wherein, the processing module is configured to output a detection trigger signal to the first power supply module, and when it is determined that a state of the fusing module is in a conduction state, output the fusing trigger signal to the second power supply module; the first power supply module is configured to receive the detection trigger signal, and output a first power supply signal to the fusing module for a state detection of the fusing module; the second power supply module is configured to receive the fusing trigger signal and output a second power supply signal to the fusing module for keeping the fusing module in a disconnected state. The invention can distinguish and identify the disposable consumables, thereby effectively avoiding the secondary use of the consumables, thereby avoiding the safety risks that may be caused by the secondary use, and has high stability and low cost.

The consumable management method and the vascular calcification treatment device provided by the present invention belong to the same inventive concept as the consumable management system provided by the present invention, and therefore have the same beneficial effects.

### DESCRIPTION OF THE DRAWINGS

The accompanying drawings described here are used to provide a further understanding of the present invention, and constitute a part of the application. The schematic embodiments of the present invention and their descriptions are used to explain the present invention, and do not constitute improper limitations to the present invention.
FIG.1 is a structural block diagram of an existing vascular calcification treatment device.
FIG.2 is a schematic structural diagram of a consumable management system provided by an embodiment of the present invention.
FIG.3 is a schematic structural diagram of a fusing module provided by an embodiment of the present invention.
FIG.4 is a schematic diagram of the specific structure and connection of the first power supply module and the second power supply module provided by an embodiment of the present invention.
FIG.5 is a schematic structural diagram of a consumable management system provided by another embodiment of the present invention.
FIG. 6 is a schematic flowchart of a consumable management method provided by an embodiment of the present invention.
FIG.7 is a schematic structural diagram of a vascular calcification treatment device according to an embodiment of the present invention.
FIG.8 is a schematic diagram of the workflow of the vascular calcification treatment device according to an embodiment of the present invention.

### Optimal Embodiment Of The Invention

In order to enable those skilled in the art to better understand the technical solutions of the present invention, the technical solutions in the embodiments of the present invention will be clearly and completely described below in conjunction with the drawings in the embodiments of the present invention. Apparently, the described embodiments are only some of the embodiments of the present invention, not all of them. All other embodiments obtained by persons of ordinary skill in the art based on the embodiments of the present invention without creative efforts shall fall within the protection scope of the present invention.

It should be noted that the terms "first" and "second" in the specification, Claims and above-mentioned drawings of the present invention are used to distinguish similar objects, but not necessarily used to describe a specific order or sequence. It is to be understood that the data so used are interchangeable under appropriate circumstances such that the embodiments of the invention described herein can be practiced in sequences other than those illustrated or described herein. Furthermore, the terms "comprising" and "having" and any variations thereof, are intended to cover a non-exclusive inclusion, for example, a process, method, system, product or device comprising a series of steps or elements is not necessarily limited to those explicitly listed steps or units, but may include other steps or units not expressly listed or inherent to the process, method, product or apparatus.

It should be understood that although the steps in the flow chart in the embodiment of the present application are displayed sequentially according to the arrows, these steps are not necessarily executed in sequence according to the arrows. Unless otherwise specified in this paper, the execution of these steps is not strictly limited to the order, and it can be executed in other order. Moreover, at least some of the steps in the figure may include multiple sub-steps or multiple stages, these sub-steps or stages are not necessarily executed at the same time, but may be executed at different times, and the execution order is not necessarily sequential Instead, it can be performed alternately or alternately with at least a part of other steps or sub-steps or stages of other steps.

Unless otherwise defined, all technical and scientific terms used herein have the same meaning as commonly understood by one of ordinary skill in the technical field of the invention. The terms used herein in the description of the present invention are for the purpose of describing specific embodiments only, and are not intended to limit the present invention.

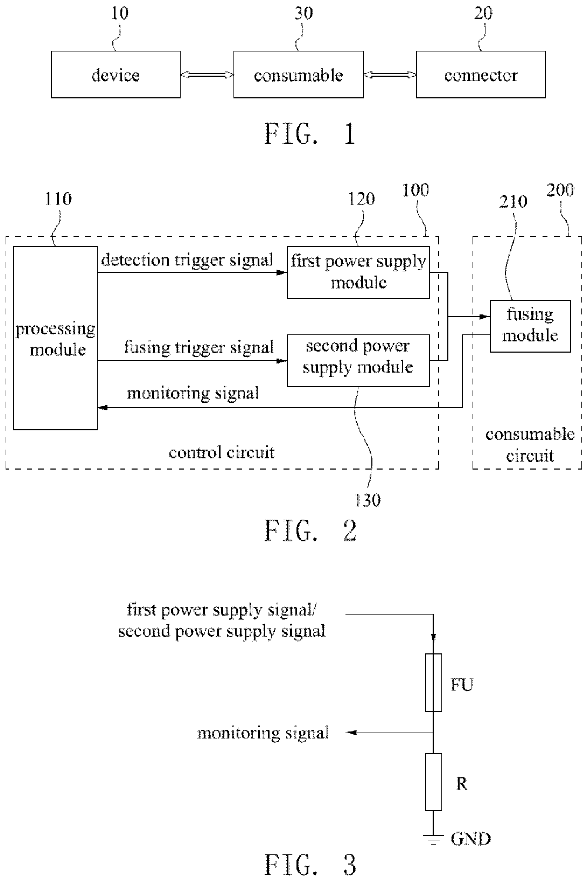

Before describing specific embodiments, it should be noted that the present application can be used for the management of various disposable consumables, and can accurately distinguish and identify new consumables and old consumables, such as consumable management for shock wave treatment device used to treat vascular calcification. Please refer to FIG. 1, which is a structural block diagram of an existing vascular calcification treatment device. As shown in FIG. 1, the existing vascular calcification treatment device includes a device 10, a consumable 20 and a connector 30, and the device 10 is connected to the consumable 20 through the connector 30. During treatment, the consumable 20 is delivered to the vascular site to be treated through the guide wire, and the high-voltage pulse generated by the device 10 is sent to the electrode of the consumable 20 through the connector 30, and shock waves are generated through the consumable electrode to fragment the calcified tissue.

For the above-mentioned devices that use non-reusable consumables, the consumables cannot be distinguished and identified, and the problem of reusable consumables will arise. However, the consumable management system provided by this application can be set on such device for consumable management, distinguish and identify disposable consumables, effectively avoid the secondary use of consumables and thus avoid possible safety risks caused by secondary use, and it is high stable and low cost.

Please refer to FIG. 2, which is a schematic structural diagram of a consumable management system provided by an embodiment of the present invention. As shown in FIG. 2, the consumable management system includes: a control circuit 100 and a consumable circuit 200, the control circuit 100 includes a processing module 110, a first power supply module 120, and a second power supply module 130, the consumable circuit 200 includes a fusing module 210, and the consumable circuit 200 is arranged in the consumable, wherein,
the processing module 110 is configured to output a detection trigger signal to the first power supply module 120, and output a fusing trigger signal to the second power supply module 130 when determining that a state of the fusing module 210 is a connected state;
the first power supply module 120 is configured to receive the detection trigger signal and output a first power supply signal to the fusing module 210 for a state detection of the fusing module 210;
the second power supply module 130 is configured to receive the fusing trigger signal and output a second power supply signal to the fusing module 210 for keeping the fusing module 210 in a disconnected state.

Specifically, the control circuit 100 and the consumable circuit 200 may be connected through a connector, the fusing module 210 may be disposed at the consumable, the processing module 110, the first power supply module 120 and the second power supply module 130 may be disposed at the device, and the processing module 110 may be microcontroller. When the consumable is connected to the device, the control circuit 100 is connected to the consumable circuit 200, and the component of the consumable is connected to the corresponding equipment of the device (for example, the consumable electrode in the vascular calcification treatment device is connected to the output end of the booster circuit on the device), the processing module 110 outputs a detection trigger signal to the first power supply module 120, and the first power supply module 120 outputs the first power supply signal to the fusing module 210 after receiving the detection trigger signal, so as to be used for state detection of the fusing module 210, that is, to determine whether the fusing module 210 is in a disconnected state. When the processing module 110 determines that the state of the fusing module 210 is the connected state, that is, when the fusing of the fusing module 210 has not occurred yet, that is, the consumable is a new consumable, the processing module 110 then outputs a fusing trigger signal to the second power supply module 130, the second power supply module 130. The second power supply module 130 outputs a second power supply signal to the fusing module 210 after receiving the fusing trigger signal, so as to make the fusing module 210 in the disconnected state, that is, making the fusing of the fusing module 210 occurring. When the processing module 110 determines that the state of the fusing module 210 is disconnected after the consumable is connected and has not yet performed fusing processing, that is, when fusing has occurred, it is determined that the consumable is an old consumable, and there is no need to send a fusing trigger signal.

It should be noted that, after the processing module 110 determines the state of the fusing module 210, the processing module 110 stops outputting the detection trigger signal.

In one embodiment, the processing module 110 controls the second power supply module 130 to output the second power supply signal to the fusing module 210 when determining that the state of the fusing module 210 is connected state and triggering pulse treatment, so as to make the fusing module 210 in the disconnected state.

Specifically, in order to avoid misdiagnosing the new consumable that has been connected but not used for treatment as the old consumable, and the problem of fusing the fusing module 210 occurs. For example, in the vascular calcification treatment device, the consumable is connected to the device, but the device does not send high voltage pulse to the consumable electrode of the consumable, that is, the consumable as not been used for treatment in fact, but the consumable is misjudged as an old consumable. In the present embodiment, when the processing module 110 determines that the state of the fusing module 210 is in the connected state and the processing module 110 triggers pulse treatment (that is, when the processing module 110 triggered the pulse treatment is detected or the processing module 110 sent the pulse treatment signal is detected), the processing module 110 outputs the trigger fusing signal to the second power supply module to control the second power supply module 130 to output the second power supply signal to the fusing module 210 to make the fusing module 210 in the disconnected state.

In one embodiment, after the processing module 110 output the fusing trigger signal to the second power supply module 130, so that the second power supply module 130 outputs the second power supply signal to the fusing module 210, so that the fuse module 210 is in the disconnected state, the processing module 110 outputs the detection trigger signal to the first power supply module 120 again. So that the first power supply module 120 outputs the first power supply signal to the fuse module 210 to determine whether the fuse module 210 is in the disconnected state.

### Embodiments Of The Invention

Please refer to FIG. 3, which is a schematic structural diagram of a fusing module provided by an embodiment of the present invention. As shown in FIG. 3, in one embodiment, the fusing module 210 includes a fuse FU and a resistor R, the first end of the fuse FU is configured to receive the first power supply signal or the second power supply signal, and the second end of the fuse FU is grounded through resistor R.

Wherein, the processing module 110 is connected to the second end of the fuse FU, and is used for obtaining a monitoring signal, which is used for judging the state of the fusing module 210.

Specifically, when the first power supply signal is output to the fusing module 210, if the consumable is a new consumable at this time, the first power supply signal passes through the fuse FU and the resistor R to the power ground, and the voltage signal amplitude of the first power supply signal is low, and the current flowing through the fuse FU cannot fuse the fuse FU. At this time, the monitoring signal obtained by the processing module 110 is at a high level, and the processing module 110 considers it to be a new consumable after detecting the high-level signal. If the consumable is a used consumable, the fuse FU in the fusing module 210 has been fused, and the current cannot flow through the fuse FU, and the monitoring signal obtained by the processing module 110 is at a low level. Therefore, the use state of the consumable can be distinguished by monitoring the difference between the high level and low level of the signal.

In one embodiment, after the detection of the consumable is completed, the processing module 110 no longer detects the monitoring signal, and at the same time, the processing module 110 controls the first power supply module 120 to stop outputting.

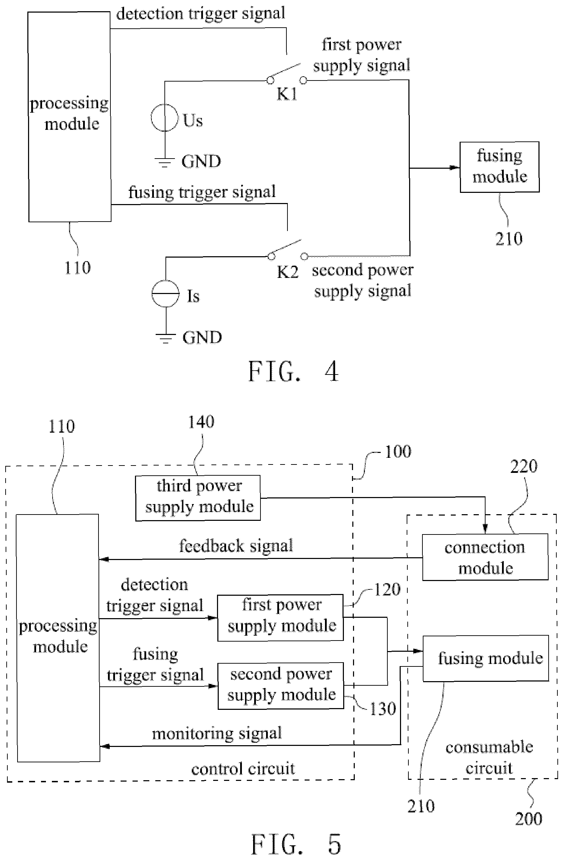

Please refer to FIG. 4. FIG. 4 is a specific structure and connection schematic diagram of a first power supply module and a second power supply module provided by an embodiment of the present invention. As shown in FIG. 4, in one embodiment, the first power supply module 120 includes a first switch K1 and a voltage source Us, and the second power supply module 130 includes a second switch K2 and a current source Is.

Wherein, the first switch K1 switches on the voltage source Us and the fusing module 210 when receiving the detection trigger signal, so that the voltage source Us outputs the first power supply signal to the fusing module 210. When the second switch K2 receives the fusing trigger signal, it switches on the current source Is and the fusing module 210, so that the current source Is outputs the second power supply signal to the fusing module 210.

Please refer to FIG. 5, which is a schematic structural diagram of a consumable management system provided by another embodiment of the present invention. As shown in FIG. 5, in one embodiment, the control circuit 100 further includes a third power supply module 140, and the consumable circuit 200 further includes a connection module 220, and the connection module 220 is configured to connect the third power supply module 140 and the processing module 110.

Wherein, the processing module 110 outputs a detection trigger signal to the first power supply module 120 when receiving a feedback signal corresponding to the third power supply signal output by the third power supply module 140 through the connection module 220.

Specifically, the connection module 220 may include, for example, a wire for connecting the output terminal of the third power supply module 140 and the feedback signal pin of the processing module 110, and the connection module 220 provided at the consumable is connected to the third power supply module 140 and the processing module 110, the pin of the processing module 110 receive a feedback signal corresponding to the third power supply signal output by the third power supply module 140, and the processing module 110 determines that the consumable has been connected to the device according to the feedback signal. For example, when the consumable is not connected, the feedback signal is a low-level signal, and after the consumable is connected, the feedback signal will be a high-level signal. Similarly, after the consumable is disconnected from the device, the feedback signal pin will become low level. After judging that the consumable is connected, the device starts to detect the state of the fusing module 210 in the consumable, and distinguishes whether the connected consumable is a new or an used.

In one embodiment, the processing module 110 is further configured to decrement the remaining treatment number of the consumable according to the pulse treatment control signal.

Specifically, the processing module 110 outputs a fusing trigger signal to the second power supply module, so as to control the fusing module 210 to set a remaining treatment number to an effective maximum after it is confirmed that the fusing module 210 is effectively fusing. The device can perform pulse treatment according to the displayed remaining treatment number. With each pulse treatment of the user, that is, when the processing module 110 receives the pulse treatment control signal, the remaining treatment number is reduced by one until the remaining treatment number is reduced to zero.

In one embodiment, the control circuit 100 further includes a backup power supply, which is configured to supply power to the processing module 110 when the processing module 110 is in a low power consumption state.

Specifically, after the main power supply of the device that supplies power to the processing module 110 is turned off, the processing module 110 triggers a low power consumption state, and the backup battery supplies power to the processing module 110. The processing module 110 is not completely powered off, and the remaining treatment number of the consumable can still be memorized, so that when the consumable is used again, the processing module 110 can continue to perform pulse treatment according to the remaining treatment number.

In one embodiment, the control circuit 100 further includes an alarm module, and the processing module 110 is connected to the alarm module, and is configured to control the alarm module to give an alarm prompt when the remaining treatment number of the consumable is 0.

Specifically, the alarm module can be a buzzer and/or an LED light, etc.

In summary, the consumable end management system provided by the embodiment of the invention can distinguish and identify the disposable consumable so as to effectively avoid the secondary use of consumables, and then avoid the security risks that may be caused by the secondary use, and also carry out effective security control of the use of consumables, with high stability and low cost.

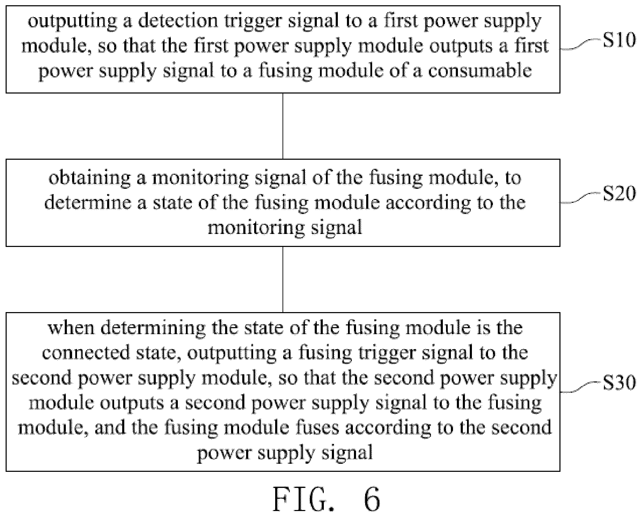

Based on the same inventive concept, an embodiment of the present invention also provides a consumable management method. Please refer to FIG. 6, which is a schematic flowchart of a consumable management method provided by an embodiment of the present invention. As shown in FIG. 6, the methods include:
S10. outputting a detection trigger signal to a first power supply module, so that the first power supply module outputs a first power supply signal to a fusing module of a consumable.

In one embodiment, step S10, outputting the detection trigger signal to the first power supply module, so that the first power supply module outputs the first power supply signal to the fusing module in the consumable, includes:
when receiving a feedback signal corresponding to a third power supply signal output by a third power supply module through the consumable, outputting the detection trigger signal to the first power supply module, so that the first power supply module outputs the first power supply signal to the fuse module in the consumable.

S20. obtaining a monitoring signal of the fusing module, to determine a state of the fusing module according to the monitoring signal.

Specifically, the state of the fusing module includes a connected state and a disconnected state, the connected state represents that the consumable is a new consumable, and the disconnected state represents that the consumable is a used consumable.

S30, when determining the state of the fusing module is the connected state, outputting a fusing trigger signal to the second power supply module, so that the second power supply module outputs a second power supply signal to the fusing module, and the fusing module fuses according to the second power supply signal.

In one embodiment, in step S30, when determining that the state of the fusing module is the connected state, outputting a fusing trigger signal to the second power supply module, so that the second power supply module outputs a second power supply signal to the fusing module, and the fusing module fuses according to the second power supply signal, including:
when determining that the state of the fusing module is the conduction state, monitoring a pulse treatment control signal;
when obtaining the pulse treatment control signal, outputting the fusing trigger signal to the second power supply module, so that the second power supply module outputs the second power supply signal to the fusing module, and the fusing module fuses according to the second power supply signal.

In one embodiment, after the step of when obtaining the pulse treatment control signal, outputting a fusing trigger signal to the second power supply module, so that the second power supply module outputs the second power supply signal to the fusing module, and the fusing module fuses according to the second power supply signal, includes:
outputting the detection trigger signal to the first power supply module, so that the first power supply module outputs the first power supply signal to the fusing module of the consumable;
obtaining a monitoring signal of the fusing module, so as to determine the state of the fusing module according to the monitoring signal;
when determining that the fusing module is in a disconnected state, setting a remaining treatment number of the consumable to an effective maximum value;
when receiving the pulse treatment control signal, determine whether the remaining treatment number of the consumable is greater than 0;
when the remaining treatment number of the consumable is greater than 0, triggering pulse treatment and decreasing the remaining treatment number of the consumable;
when the remaining treatment number of the consumable is equal to 0, not triggering the pulse treatment.

It should be noted that the remaining treatment number of the consumable is set to the effective maximum value, which means subtracting 1 from the default maximum value of the consumable.

In one embodiment, the consumable management method further includes:
when determining that the state of the fusing module is disconnected, monitoring the pulse treatment control signal;
when receiving the pulse treatment control signal, determine whether the remaining treatment number of the consumable is greater than 0;
when the remaining treatment number of the consumable is greater than 0, triggering the pulse treatment, and decreasing the remaining treatment number of the consumable;
when the remaining treatment number of the consumable is equal to 0, not triggering the pulse treatment.

In one embodiment, after the step of when the remaining treatment number of the consumable is equal to 0, not triggering the pulse treatment, further include:
making an alarm prompt.

It should be noted that, for parts that are not described or described in detail in the embodiment of the method, please refer to the description of the foregoing embodiments, and details are not repeated here.

In summary, the consumable management method provided by the embodiment of the present invention can distinguish and identify the disposable consumable, effectively avoid the secondary use of the consumable, thereby avoiding the safety risk that may be caused by the secondary use, and can perform effective security control for the use of the consumable, with high stability and low cost.

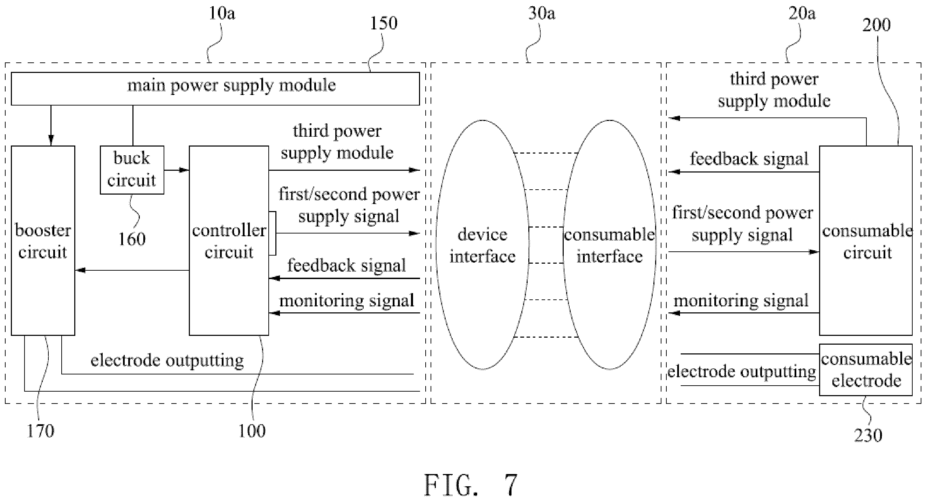

Based on the same inventive concept, an embodiment of the present invention also provides a consumable management method. Please refer to FIG. 7, which is a schematic structural diagram of a vascular calcification treatment device according to an embodiment of the present invention. As shown in FIG. 7, the device includes: a device 10a, a consumable 20a, and a connector 30a, the device is connected to the consumable through the connector, and the consumable 20a includes a consumable electrode 230 and the consumable circuit 200 described in any of the above-mentioned embodiments. The device 10a includes a main power supply module 150, a buck circuit 160, a booster circuit 170, and the control circuit 100 in any of the above-mentioned implementation manners; wherein,
the main power supply module 150 is connected to the booster circuit 170, and is connected to the processing module 110, the first power supply module 120 and the second power supply module 130 through the buck circuit 160 to power supply;
the processing module 110 is connected to the booster circuit 170, and is configured to control the booster circuit 170 to output a pulse signal;
the booster circuit 170 is connected to the consumable electrode 230, to form a high voltage discharge loop to release the pulse signal.

In one embodiment, the main power supply module 150 is connected to the third power supply module 140 through the buck circuit 160 to supply power.

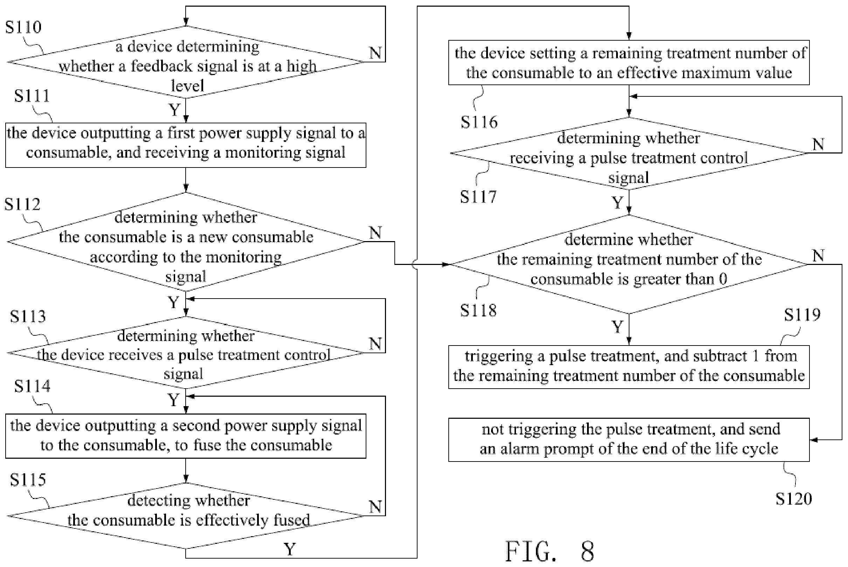

In order to describe the technical solution of the present invention more clearly, the workflow of the vascular calcification treatment device shown in FIG. 7 will be described in detail below. Please refer to FIG. 8 in conjunction with FIG. 8, which is a schematic workflow diagram of a vascular calcification treatment device provided by an embodiment of the present invention. As shown in FIG. 8, the workflow of the vascular calcification treatment device includes the following steps:
S 110: the device determining whether the feedback signal is at a high level;
   when the feedback signal is at a low level, returning to step S110;
   when the feedback signal is at a high level, entering step S111: the device outputting a first power supply signal to the consumable, and receiving a monitoring signal;
S112: determining whether the consumable is a new consumable according to the monitoring signal;
   when determining that the consumable is an old consumable, go to step S117;
   when determining that the consumable is a new consumable, enter step S113: determining whether the device receives a pulse treatment control signal;
   returning to step S113 when the device does not receive the pulse treatment control signal;
   when the device receives the pulse treatment control signal, enter step S114: the device outputting a second power supply signal to the consumable, to fuse the consumable.
Step S 115: detecting whether the consumable is effectively fused;
   when the consumable is not effectively fused, return to step S114;
   when the consumable is effectively fused, enter step S116: the device setting the remaining treatment number of the consumable to the effective maximum value;
Step S117: determining whether receiving a pulse treatment control signal;
   when not receiving the pulse treatment control signal, return to step S117;
   when receiving the pulse treatment control signal, enter step S118: determine whether the remaining treatment number of the consumable is greater than 0;
   when the remaining treatment number of the consumable is greater than 0, enter step S119: triggering the pulse treatment, and subtract 1 from the remaining treatment number of the consumable;
   when the remaining treatment number of the consumable is not greater than 0, enter step S 120: not triggering the pulse treatment, and send an alarm prompt of the end of the life cycle.

It should be noted that, for parts that are not described or described in detail in the embodiment of the method, please refer to the description of the foregoing embodiments, and details are not repeated here.

### Industrial Applicability

In summary, the vascular calcification treatment equipment provided by the embodiment of the invention can distinguish and identify the disposable consumable, so as to effectively avoid the secondary use of the consumable, thereby avoiding the safety risks that may be caused by the secondary use, and can effectively control the safety of the use of the consumable, with high stability and low cost.

The above is only a better embodiment of the invention, and is not a restriction on the invention in any form. Although the invention has been disclosed as a better embodiment, it is not used to limit the invention. Any technical personnel familiar with the profession, without deviating from the scope of the technical scheme of the invention, may make a slight change or modification to the equivalent embodiment of the equivalent change by using the technical content disclosed above. However, any simple modification, equivalent change and modification to the above embodiment according to the technical essence of the invention, without deviating from the content of the technical scheme of the invention, still belongs to the scope of the technical scheme of the invention.

## Claims

1. A consumable management system is **characterized in that** it comprises a control circuit and a consumable circuit, the control circuit comprises a processing module, a first power supply module and a second power supply module, the consumable circuit comprises a fusing module, the consumable circuit is arranged in a consumable, wherein,
the processing module is configured to output a detection trigger signal to the first power supply module, and output a fusing trigger signal to the second power supply module when determining that a state of the fusing module is a connected state;
the first power supply module is configured to receive the detection trigger signal and output a first power supply signal to the fusing module for a state detection of the fusing module;
the second power supply module is configured to receive the fusing trigger signal and output a second power supply signal to the fusing module for keeping the fusing module in a disconnected state.

2. The consumable management system according to claim 1, is **characterized in that**, the processing module outputs the fusing trigger signal to the second power supply when determining that the state of the fusing module is a connected state and the processing module triggers pulse treatment, to control the second power supply module to output the second power supply signal to the fusing module, so as to keep the fusing module in a disconnected state.

3. The consumable management system according to claim 1, is **characterized in that**, the fusing module comprises a fuse and a resistor, a first end of the fuse is configured to receive the first power supply signal or the second power supply signal, and a second end of the fuse is grounded through the resistor;
the processing module is connected to the second end of the fuse, and configured to obtain a monitoring signal to determine the state of the fusing module according to the monitoring signal.

4. The consumable management system according to claim **1,** is **characterized in that**, the first power supply module comprises a first switch and a voltage source, and the second power supply module comprises a second switch and a current source;
when the first switch receives the detection trigger signal, the first switch switches on the voltage source and the fusing module, so that the voltage source outputs the first power supply signal to the fusing module;
when the second switch receives the fusing trigger signal, the second switch switches on the current source and the fusing module, so that the current source outputs the second power supply signal to the fusing module.

5. The consumable management system according to claim 1, is **characterized in that**, the control circuit further comprises a third power supply module, the consumable circuit also comprises a connection module, the connection module is connected to the third power supply module and the processing module through feedback signal fines; wherein,
when the processing module receives a feedback signal corresponding to a third power supply signal output by the third power supply module through the connection module, the processing module outputs the detection trigger signal to the first power supply module.

6. A consumable management method, is **characterized in that**, the method comprises:
outputting a detection trigger signal to a first power supply module, so that the first power supply module outputs a first power supply signal to a fusing module of a consumable;
obtaining a monitoring signal of the fusing module, to determine a state of the fusing module according to the monitoring signal;
when determining the state of the fusing module is a connected state, outputting a fusing trigger signal to a second power supply module, so that the second power supply module outputs a second power supply signal to the fusing module, and the fusing module fuses according to the second power supply signal.

7. The consumable management method according to claim 6, is **characterized in that**, a step of outputting the detect trigger signal to the first power supply module so that the first power supply module outputs the first power supply signal to the fusing module of the consumable comprises:
when receiving a feedback signal corresponding to a third power supply signal output by a third power supply module through the consumable, outputting the detection trigger signal to the first power supply module, so that the first power supply module outputs the first power supply signal to the fusing module of the consumable.

8. The consumable management method according to claim 6 or 7, is **characterized in that**, a step of when determining the state of the fusing module is the connected state, outputting the fusing trigger signal to the second power supply module, so that the second power supply module outputs the second power supply signal to the fusing module, and the fusing module fuses according to the second power supply signal, comprises:
when determining the state of the fusing module is connected state, monitoring a pulse treatment control signal;
when obtaining the pulse treatment control signal, outputting the fusing trigger signal to the second power supply module, so that the second power supply module outputs the second power supply signal to the fusing module, so that the fusing module fuses according to the second power supply signal.

9. The consumable management method according to claim **8,** is **characterized in that**, after a step of when obtaining the pulse treatment control signal, outputting the fusing trigger signal to the second power supply module, so that the second power supply module outputs the second power supply signal to the fusing module, so that the fusing module fuses according to the second power supply signal, comprises:
outputting the detection trigger signal to the first power supply module, so that the first power supply module outputs the first power supply signal to the fusing module of the consumable;
obtaining a monitoring signal of the fusing module to determine the state of the fusing module according to the monitoring signal;
when determining that the fusing module is in a disconnected state, setting a remaining treatment number of the consumable to an effective maximum;
when the remaining treatment number of the consumable is greater than 0, and receiving the pulse treatment control signal, triggering pulse treatment and decreasing the remaining treatment number of the consumable;
when the remaining treatment number of the consumable is equal to 0 and receiving the pulse treatment control signal, not triggering the pulse treatment.

10. A vascular calcification treatment device, is **characterized in that**, it comprises an device, a connector and the consumable as claimed in any one of the claims 1-5, the device is connected to the consumable through the connector, the consumable also comprises a consumable electrode, the device comprises a main power supply module, a buck circuit, a booster circuit and the control circuit as claimed in any one of claims **1-5;** wherein,
the main power supply module is connected to the booster circuit, and connected to the processing module, the first power supply module and the second power supply module through the buck circuit, to supply power;
the processing module is connected to the booster circuit, and is configured to control the booster circuit output a pulse signal;
the booster circuit is connected to the consumable electrode, to form a high voltage discharge loop to release the pulse signal.
